**Europäisches Patentamt**

**European Patent Office**

**Office européen des brevets**

(19)

(11) Veröffentlichungsnummer **0 019 006**

**A1**

(12) **EUROPÄISCHE PATENTANMELDUNG**

(21) Anmeldenummer: **79101475.6**

(51) Int. Cl.³: **A 61 K 31/415**

(22) Anmeldetag: **14.05.79**

(43) Veröffentlichungstag der Anmeldung:
**26.11.80 Patentblatt 80/24**

(84) Benannte Vertragsstaaten:
**AT CH FR GB IT LU NL SE**

(71) Anmelder: **CIBA-GEIGY AG**
**Patentabteilung Postfach**
**CH-4002 Basel(CH)**

(72) Erfinder: **Kane, Sydney H., Dr.**
**12 Stockade Road**
**Warren New Jersey 07060(US)**

(72) Erfinder: **Margulies, Erwin, Dr.**
**23 Regina Road**
**Morganville New Jersey 07751(US)**

(74) Vertreter: **Zumstein sen., Fritz, Dr. et al,**
**Bräuhausstrasse 4**
**D-8000 München 2(DE)**

(54) **Pharmazeutische Präparate, die als Wirkstoff ein substituiertes Pyrazolidinderivat enthalten, deren Verwendung und Verfahren zu ihrer Herstellung.**

(57) Die Erfindung betrifft Präparate, die als Wirkstoff ein substituiertes Pyrazolidindion, das 1,2-Diphenyl-4-(2-phenylthioäthyl)- pyrazolidin-3,5-dion oder ein pharmazeutisch annehmbares Salz davon mit einer Base enthalten und deren Verwendung für die Behandlung cardiovaskulärer Erkrankungen.

EP 0 019 006 A1

BEZEICHNUNG GEÄNDERT
siehe Titelseite

4-11675/CGC 858/-

Präparate, die ein substituiertes Pyrazolidinderivat als Wirkstoff
enthalten und deren Verwendung.

Die Erfindung betrifft Präparate, die als Wirkstoff ein substituiertes Pyrazolidindion, das 1,2-Diphenyl-4-(2-phenylthioäthyl)-
pyrazolidin-3,5-dion oder ein pharmazeutisch annehmbares Salz
davon mit einer Base enthalten und deren Verwendung für die Behandlung cardiovaskulärer Erkrankungen. Insbesondere betrifft die Erfindung die Verwendung des 1,2-Diphenyl-4-(2-phenylthioäthyl)-
pyrazolidin-3,5-dions und seiner Salze als die Thrombocytenaggregation hemmende Arzneimittelwirkstoffe, insbesondere zur Behandlung
thrombotischer Erkrankungen und diese oder ein pharmazeutisch
nehmbares Salz davon enthaltende, die Thrombocytenaggregation hemmende, pharmazeutischen Präparate.

1,2-Diphenyl-4-(2-phenylthioäthyl)-pyrazolidin-3,5-dion ist
bekannt und, nebst einem Verfahren zu seiner Herstellung, in Helv.
Chim. Acta 44, 232-237 (1961) beschrieben.

Ferner ist beispielsweise aus der Schweiz. Med. Wochenschrift
84, 1315-1318 (1954) an Hand tierexperimenteller Versuchsergebnisse und klinischer Versuche am Patienten bekannt, dass 1,2-Diphenyl-4-(-2-phenylthioäthyl)-pyrazolidin-3,5-dion und seine pharmazeutisch verwendbaren Salze antiphlogistisch und analgetisch wirksam sind und hiermit zur Behandlung rheumatischer Erkrankungen, insbesondere der rheumatischen Arthritis verwendet werden können. Diesen erwähnten klinischen Versuchen zufolge konnte bei etwa 2/3 der Patienten durch orale Verabreichung von 1,2-Diphenyl-4-(2-phenylthioäthyl)-pyrazolidin-3,5-dion mittels Tagesdosen von 1 bis 2 g unter Verabreichung von Einzeldosen von 0,2 g eine Besserung der Arthritis erzielt werden. Es ist weiterhin aus J. Pharmacol., 119, 418-426 (1957) bekannt, dass 1,2-Diphenyl-4-(2-phenylthioäthyl)-pyrazolidin-3,5-dion im menschlichen Organismus zum entsprechenden Sulfoxid, dem 4-(2-Benzolsulfinyl-äthyl)-1,2-diphenyl-pyrazolidin-3,5-dion (Sulfinpyrazon) metabolisiert wird. Dieser Publikation zufolge hat 4-(2-Benzolsulfinyläthyl)-1,2-diphenylpyrazolidin-3,5-dion eine ausgeprägte urikosurische Wirkung und wird als eigentlich wirksame Komponente für alle bis anhin festgestellten pharmakologischen Wirkungen des 1,2-Diphenyl-4-(2-phenylthioäthyl)-pyrazolidin-3,5-dion verantwortlich gemacht.

Ueberraschenderweise konnte nun festgestellt werden, dass 1,2-Diphenyl-4-(2-phenylthioäthyl)-pyrazolidin-3,5-dion und seine Salze als Wirksubstanzen für die Behandlung von cardiovaskulären Erkrankungen, insbesondere von Zuständen, bei denen eine abnorme Funktion der Blutplättchen den verursachenden oder begleitenden Faktor darstellt, wie für die Behandlung von thrombotischen Erkrankungen ausgezeichnet geeignet sind.

Es hat sich herausgestellt, dass die obengenannte Verbindung und ihre Salze in Versuchsanordnungen zum Nachweis einer antithrombotischen Wirkung, insbesondere in Cyclooxygenase-abhängigen Testen eine

- 3 -

bis gegen 10-mal stärkere Wirkung besitzen als Sulfinpyrazon.
Diese ausgeprägte antithrombotische Aktivität kann anhand der Arthus-
Reaktion, siehe dazu Brit. J. Pharmakology, 57, 441 p (1976), im
Dosisbereich von etwa 5-50 mg/kg p.o.

und anhand der Prostaglandin-Synthetasehemmung aus Arachidonsäure, nachgewiesen in der Versuchsanordnung
nach Prostaglandin, 7,123 (1974) in vitro im Konzentrationsbereich
von 75-750 mg/l, dokumentiert werden.

1,2-Diphenyl-4-(2-phenylthioäthyl)-pyrazolidin-3,5-
dion und seine pharmazeutisch verwendbaren Salze sind dementsprechend
ausgezeichnet zur Behandlung cardiovakulärer Erkrankungen, insbesondere zur Behandlung thrombotischer Erkrankungen, beispielsweise
bei Herzinfarktpatienten zur Verhütung eines plötzlichen Exitus
("sudden death") geeignet und können deshalb als Wirkstoff in antithrombotischen Arzneimitteln verwendet werden.

Pharmazeutisch verwendbare Salze des 1,2-Diphenyl-4-(2-
phenylthioäthyl)-pyrazolidin-3,5-dions mit Basen sind z.B. pharmazeutisch verwendbare Metallsalze oder Ammoniumsalze. Als pharmazeutisch verwendbare Metallsalze kommen insbesondere Alkali- oder
Erdalkalimetallsalze, wie z.B. das Natrium-, Kalium-, Calcium-
oder Magnesiumsalz, ferner das Zink- und Kupfersalz in Betracht.
Als pharmazeutisch verwendbare Ammoniumsalze kommen beispielsweise
Ammoniumsalze mit Ammoniak oder pharmazeutisch unbedenklichen organischen Aminen, wie z.B. gegebenenfalls substituierten Mono-,
Di- oder Trialkylaminen mit jeweils bis zu 7, vor allem bis
4 Kohlenstoffatomen im Alkyl.

– 4 –

Als Beispiele dafür seien insbesondere Diäthylamin, Aethylendiamin, Trihydroxymethylmethylamin, ferner Triäthylamin, Methylamin,
Triäthanolamin und Benzyl-trimethylamin genannt.

Die vorliegende Erfindung betrifft dementsprechend pharmazeutische Präparate und deren Verwendung, welche die erfindungsgemässe
Verbindung oder ein pharmazeutisch verwendbares Salz davon enthalten.
Bei den erfindungsgemässen pharmazeutischen Präparaten handelt es sich
um solche, die zur enteralen, wie oralen oder rektalen, sowie parenteralen Verabreichung bestimmt sind und den pharmakologischen Wirkstoff
allein oder zusammen mit einem pharmazeutisch anwendbaren Trägermaterial enthalten.

Die neuen pharmazeutischen Präparate enthalten z.B. von etwa
10 % bis etwa 95 %, vorzugsweise von etwa 20 % bis etwa 70 % des
Wirkstoffs. Erfindungsgemässe pharmazeutische Präparate sind z.B.
solche in Dosiseinheitsformen, wie Dragées, Tabletten, Kapseln oder
Suppositorien, ferner Ampullen. Ihre Einzeldosen enthalten zwischen
vorzugsweise von etwa 50 bis etwa 200 mg, vorzugsweise bis etwa 100 mg
des obengenannten Wirkstoffes. Der Wirkstoff wird in einer täglichen
Dosis bis zu 1,0 g, vorzugsweise von 0,25 bis 1,0 g, einem Patienten
von ungefähr 75 kg Körpergewicht verabreicht.

Die pharmazeutischen Präparate der vorliegenden Erfindung
werden in an sich bekannter Weise, z.B. mittels konventioneller
Misch-, Granulier- , Dragier-, Lösungs- oder Lyophilisierungsverfahren hergestellt.

So kann man pharmazeutische Präparate zur oralen Anwendung erhalten, indem man den Wirkstoff mit festen Trägerstoffen kombiniert,
ein erhaltenes Gemisch gegebenenfalls granuliert, und das Gemisch
bzw. Granulat, wenn erwünscht oder notwendig, nach Zugabe von
geeigneten Hilfsstoffen, zu Tabletten oder Dragée-Kernen verarbeitet. Geeignete Trägerstoffe sind insbesondere Füllstoffe, wie

- 5 -

Zucker, z.B. Lactose, Saccharose, Mannit oder Sorbit, Cellulosepräparate und/oder Calciumphosphate, z.B. Tricalciumphosphat
oder Calciumhydrogenphosphat, ferner Bindemittel, wie Stärkekleister
und Verwendung z.B. von Mais-, Weizen-, Reis- oder Kartoffelstärke,
Gelatine, Traganth, Methylcellulose und/oder Polyvinylpyrrolidon,
und/oder, wenn erwünscht, Sprengmittel, wie die obgenannten Stärken,
ferner Carboxymethylstärke, quervernetztes Polyvinylpyrrolidon,
Agar, Alginsäure oder ein Salz davon, wie Natriumalginat, Hilfsmittel sind in erster Linie Fliessregulier- und Schmiermittel,
z.B. Kieselsäure, Talk, Stearinsäure oder Salze davon, wie Magnesium-
oder Calciumstearat, und/oder Polyäthylenglykol. Dragée-Kerne werden mit geeigneten, gegebenenfalls Magensaft-resistenten Ueberzügen
versehen, wobei man u.a. konzentrierte Zuckerlösungen, welche gegebenenfalls arabischen Gummi, Talk, Polyvinylpyrrolidon, Polyäthylenglycol und/oder Titandioxid enthalten. Lacklösungen in geeigneten
organischen Lösungsmitteln oder Lösungsmittelgemischen oder, zur
Herstellung von Magensaft-resistenten Ueberzügen, Lösungen von geeigneten Cellulosepräparaten, wie Acetylcellulosephthalat oder
Hydroxypropylmethylcellulosephthalat, verwendet. Den Tabletten
oder Dragée-Ueberzügen können Farbstoffe oder Pigmente, z.B. zur
Identifizierung oder zur Kennzeichnung verschiedener Wirkstoffdosen,
beigefügt werden.

Weitere oral anwendbare pharmazeutische Präparate sind
Steckkapseln aus Gelatine, sowie weiche, geschlossene Kapseln
aus Gelatine und einem Weichmacher, wie Glycerin oder Sorbitol.
Die Steckkapseln können den Wirkstoff in Form eines Granulates,
z.B. im Gemisch mit Füllstoffen, wie Lactose, Bindemitteln, wie
Stärken, und/oder Gleitmitteln, wie Talk oder Magnesiumstearat,
und gegebenenfalls von Stabilisatoren, enthalten. In weichen Kapseln
ist der Wirkstoff vorzugsweise in geeigneten Flüssigkeiten, wie
fetten Oelen, Paraffinöl oder flüssigen Polyäthylenglykolen, gelöst oder suspendiert, wobei ebenfalls Stabilisatoren zugefügt
sein können.

- 6 -

Als rektal anwendbare pharmazeutische Präparate kommen z.B.
Suppositorien in Betracht, welche aus einer Kombination des
Wirkstoffs mit einer Suppositoriengrundmasse bestehen. Als Suppositoriengrundmasse eignen sich z.B. natürliche oder synthetische
Triglyceride, Paraffinkohlenwasserstoffe, Polyäthylenglykole
oder höhere Alkanole. Ferner können auch Gelatine-Rektalkapseln
verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in
Frage.

Zur parenteralen Verabreichung eignen sich in erster Linie
wässrige Lösungen eines Wirkstoffs in wasserlöslicher Form, z.B.
eines wasserlöslichen Salzes, ferner Suspensionen des Wirkstoffs,
wie entsprechende ölige Injektionssuspensionen, wobei man geeignete
lipophile Lösungsmittel oder Vehikel, wie fette Oele, z.B. Sesamöl,
oder synthetische Fettsäureester, z.B. Aethyloleat oder Triglyceride, verwendet.oder wässrige Injektionssuspensionen, welche
viskositätserhöhende Stoffe, z.B. Natriumcarboxymethylcellulose,
Sorbit und/oder Dextran und gegebenenfalls auch Stabilisatoren
enthalten.

Die nachfolgenden Beispiele illustrieren die oben beschriebene
Erfindung; sie sollen diese jedoch in ihrem Umfang in keiner Weise einschränken.

Beispiel 1: Herstellung von 1000 mit Zucker überzogenen Tabletten mit einem Gehalt von je 200 mg der aktiven Substanz.

Bestandteile des Kerns

| | | |
|---|---|---|
| 1,2-Diphenyl-4-(2-phenylthioäthyl)-pyrazolidin-3,5-dion | 200 | g |
| Maisstärke | 58 | g |
| Milchzucker | 15,3 | g |
| Stearinsäure | 6 | g |
| Gelatine | 6 | g |
| Glycerin | 1,7 | g |
| Talkpulver | 12 | g |
| Magnesiumstearat | 1 | g |
| wasserfreies Aethanol | q.s. | |
| Wasser | q.s. | |

Verfahren: Der Wirkstoff, die Stärke und der Milchzucker werden in einem geeigneten Mischer gut vermischt. Die Stearinsäure wird in heissem Aethanol, und die Gelatine und das Glycerin werden in heissem Wasser gelöst. Beide Lösungen werden zu dem obigen Pulvergemisch gegeben und es wird ein Granulat hergestellt. Das erhaltene Granulat wird getrocknet, durch ein Sieb mit 1,2 mm Maschenweite getrieben, mit dem Talkpulver und Magnesiumstearat vermischt und zu Tablettenkernen von 300 mg gepresst.

Bestandteile des Ueberzugs:

| | | |
|---|---|---|
| Schellack-Lösung Bestandteile | 1,40 | g |
| Talkpulver | 22 | g |
| Titandioxid | 2,50 | g |
| Rizinusöl | 0,30 | g |
| Rohrzucker | 119,06 | g |
| Polyvinylpyrrolidon | 1,56 | g |
| Mikrokristalline Cellulose | 1,04 | g |
| Polyäthylenglykol 6000 | 1,04 | g |

- 8 -

| | | |
|---|---:|---|
| Rohrzucker mit 3 % Maisstärke | 1,02 | g |
| Carnaubawachs | 0,07 | g |
| wasserfreies Aethanol | q.s. | |
| Wasser | q.s. | |

<u>Verfahren:</u> Eine versiegelnde Lösung wird aus der Schallacklösung, Aethanol und Rizinusöl in einer geeigneten Ueberzugspfanne vorbereitet. Die Tablettenkerne werden, wenn nötig unter Trocknen, mit Talk und Titandioxid bestäubt. Zur Beschichtung wird eine genügende Menge des Rohrzuckersyrups in Wasser verwendet, die Tablettenkerne werden dann mit dem Rohrzucker-Stärke Gemisch bestäubt und gegebenenfalls getrocknet. Eine genügende Menge vom Grundsyrup, der aus Polyäthylenglykol, Aethanol, Polyvinylpyrrolidon, Beschichtungssyrup, Cellulose, Titandioxid und Talk vorbereitet ist, wird angewendet, und die Kerne werden, falls nötig, getrocknet. Nachher werden der Beschichtungssyrup und der Titandioxyd-Farbstoff-Syrup mehrmals angewendet, falls nötig unter Trocknen zwischen den einzelnen Beschichtungen. Die erhaltenen überzogenen 450 mg schweren Tabletten werden schliesslich mit Carnaubawachs poliert und falls nötig, mit Markenbezeichnung versehen.

<u>Beispiel 2:</u> Herstellung von 1000 Kapseln mit einem Gehalt von je 200 mg der aktiven Substanz.

<u>Bestandteile</u>

| | | |
|---|---:|---|
| 1,2-Diphenyl-4-(2-phenylthioethyl)-pyrazolidin-2,5-dion | 200 | g |
| Maisstärke | 29 | g |
| Gelatine | 6,5 | g |
| Milchzucker | 90 | g |
| Magnesiumstearat | 3,5 | g |
| Stearinsäure | 3 | g |
| Talkpulver | 3,5 | g |
| Isopropanol | q.s. | |
| Wasser | q.s. | |

- 9 -

Verfahren:  Der Wirkstoff, 20 g Stärke und der Milchzucker werden in einem geeigneten Mischer vermischt. Die Stearinsäure wird in heissem Isopropanol und die Gelatine in heissem Wasser gelöst. Beide Lösungen werden zu den Pulvern gegeben und das erhaltene Granulat, falls nötig, mit Wasser befeuchtet. Die Masse wird in einer Zerkleinerungsmaschine behandelt, getrocknet und das Granulat mit dem Rest von 9 g Stärke, mit Talk und Magnesiumstearat vermischt. Das fertige Gemisch wird in harte Gelatinekapseln Nr. 1 gefüllt und, wenn nötig, mit essbarer Tinte beschriftet.

Beispiel 3: Um 1000 Kapseln mit je 100 mg Wirkstoffgehalt herzustellen, mischt man 100 g Wirkstoff mit 173,0 g Lactose, befeuchtet die Mischung gleichmässig mit einer wässrigen Lösung von 2,0 g Gelatine und granuliert sie durch ein geeignetes Sieb (z.B. Sieb III nach Ph.Helv. V). Das Granulat mischt man mit 10,0 g getrockneter Maisstärke und 15,0 g Talk und füllt es gleichmässig in 1000 Hartgelatinekapseln der Grösse 1.

Beispiel 4: Zur parenteralen Verabreichung eignen sich in erster Linie Injektionslösungen der oben beschriebenen Salze, insbesondere Alkalimetallsalze.

Injektionslösungen eines Natriumsalzes des Wirkstoffes werden beispielsweise wie folgt hergestellt:

| | | |
|---|---|---|
| Wirkstoff | 10,0 | g |
| Benzylalkohol, pH | 1,0 | g |
| Natriumsulfit, purum | 0,1 | g |
| EDTA, Dinatriumsalz | 0,2 | g |
| Vitamin C | 0,3 | g |

werden mit Natronlauge auf den PH 8,6 gestellt und mit pyrogenfreiem Wasser auf 100 ml ergänzt. Die Lösung wird abfiltriert und in Ampullen zu à 1 ml abgefüllt und sterilisiert. Eine Ampulle à 1 ml enthält 100 mg Wirkstoff.

Patentansprüche

1. Pharmazeutische Präparate, enthaltend das 1,2-Diphenyl-4-
(2-phenylthioäthyl)-pyrazolidin-3,5-dion oder ein pharmazeutisch
annehmbares Salz davon mit einer Base in Einzeldosen, die sich
zur Verabreichung einer Tagesdosis von bis zu 1 g Wirkstoff eignen.

2. Pharmazeutische Präparate gemäss Anspruch 1, die sich zur oralen
Verabreichung eignen.

3. Pharmazeutische Präparate gemäss Ansprüchen 1 und 2, die
sich zur Verabreichung in Tabletten mit einem Gehalt von 50
bis 200 mg an freiem Wirkstoff eignen.

4. Pharmazeutische Kapseln gemäss Ansprüchen 1 und 2, die sich
zur Verabreichung in Kapseln mit einem Gehalt von 50 bis 200 mg an
freiem Wirkstoff eignen.

5. Verwendung des 1,2-Diphenyl-4-(2-phenylthioäthyl)-pyrazolidin-
3,5-dions oder eines pharmazeutisch annehmbaren Salzes mit
einer Base zur Behandlung cardiovaskulärer Erkrankungen.

6. Verwendung des 1,2-Diphenyl-4(-phenylthioäthyl)-pyrazolidin-
3,5-dions oder eines pharmazeutisch annehmbaren Metallsalzes
zur Behandlung von thrombotischen Erkrankungen.

7. Verwendung des 1,2-Diphenyl-4-(2-phenylthioäthyl)-pyrazolidin-
3,5-dions oder eines pharmazeutisch annehmbaren Metallsalzes
zur Behandlung von Zuständen, bei denen eine abnorme Funktion der Blutplättchen den verursachenden oder begleitenden Faktor darstellt.

8. Verwendung des 1,2-Diphenyl-4-(2-phenylthioäthyl)-pyrazoli-
din-3,5-dions oder eines pharmazeutisch akzeptablen Metallsalzes gemäss Ansprüchen 5 bis 7, dadurch gekennzeichnet, dass die Verabreichung enteral oder parenteral erfolgt.

9.    Verwendung des 1,2-Diphenyl-4-(2-phenylthioäthyl)-pyrazolidin-3,5-dions oder eines pharmazeutisch akzeptablen Metallsalzes gemäss Ansprüchen 5 bis 7, dadurch gekennzeichnet, dass die Verabreichung oral erfolgt.

10.    Verwendung des 1,2-Diphenyl-4-(2-phenylthioäthyl)-pyrazolidin-3,5-dions oder eines pharmazeutisch akzeptablen Metallsalzes gemäss Ansprüchen 5 bis 7, dadurch gekennzeichnet, dass die Verabreichung in Einzeldosen von 50 bis 200 mg in Form von Tabletten, Kapseln oder Dragées erfolgt.

11.    Verfahren zur Herstellung von pharmazeutischen Präparaten, dadurch gekennzeichnet, dass man 1,2-Diphenyl-4-(2-phenylthioäthyl)-pyrazolidin-3,5-dion oder ein pharmazeutisch verwendbares Salz davon mit einem Trägermaterial vermischt und das Gemisch zu Präparaten verarbeitet, die den Wirkstoff in Einzeldosen enthalten, die sich zur Verabreichung einer Tagesdosis von bis zu 1 g Wirkstoff eignen.

## EUROPÄISCHER TEILRECHERCHENBERICHT,
der nach Regel 45 des Europäischen Patentübereinkommens für das weitere Verfahren als
europäischer Recherchenbericht gilt

Europäisches Patentamt

Nummer der Anmeldung

EP 79 10 1475

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | DE - C - 903 578 (J.R. GEIGY AG)  * Seite 1, Zeile 1 - Seite 2, Zeile 2; Seite 4, Zeilen 19-26; Zeilen 109-110 *  -- | 1-4,11 |
| X | FR - A - 2 296 430 (CIBA-GEIGY AG)  * Seite 18, Zeilen 1-29; Seite 1, Zeilen 21-34 *  -- | 1-4,11 |
| X | MARTIN NEGWER: "Organisch-Chemische Arzneimittel und ihre Synonima" (Akademie-Verlag, Berlin) 1978 Nr. 5061  -- | 1-4,11 |
| D | SCHWEIZ.MED.WOCHENSCHRIFT, Band 84, 1954, Seiten 1315-1318 G. WILHELMI: "Über die pharmakologischen und klinischen Eigenschaften von G 25671 (Geigy Basel) einem neuen Präparat aus der Pyrazolidinreihe"  ./. | 1-4,11 |

### KLASSIFIKATION DER ANMELDUNG (Int Cl ³)

A 61 K 31/415

### RECHERCHIERTE SACHGEBIETE (Int Cl ³)

A 61 K 31/415
C 07 D 231/36
       231/32

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-4,11

Unvollständig recherchierte Patentansprüche:

Nicht recherchierte Patentansprüche: 5-10 Verfahren zur chirurgischen oder

Grund für die Beschränkung der Recherche:

therapeutischen Behandlung des menschlichen oder tierischen Körpers. (Siehe Art. 52(4) des Europäischen Patentübereinkommens).

### KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Bdatum der Recherche | Prüfer |
|---|---|---|
| Den Haag | 10-01-1980 | BRINKMANN |

EPA Form 1505.1  06.78

Europäisches
Patentamt

**EUROPÄISCHER TEILRECHERCHENBERICHT**

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | * Seite 1317, Versuch I; Seite 1318, Zusammenfassung: 4 * | | |
| | -- | | |
| D | J. PHARMACOL., Band 119, 1957, Seiten 418-426 J.J. BURNS: "A potent new urico-suric agent, the sulfoxide meta-bolite of the phenylbutazone ana-logue, G-25671" | 1-4,11 | |
| | * Seite 418, results, Seite 423, Tabelle 4 * | | RECHERCHIERTE SACHGEBIETE (Int. Cl.³) |
| | ----- | | |